# EUROPEAN PATENT SPECIFICATION

(11) **EP 3 054 000 B1**
(45) Date of publication and mention of the grant of the patent: **27.12.2017**
(21) Application number: 16152901.1
(22) Date of filing: 27.01.2016
(51) Int. Cl.: C12M 1/107, C12M 1/00, C12M 1/08, C12M 1/34

(54) **GAS-LIFT DIGESTER FOR THE ANAEROBIC DIGESTION OF WASTE COMING FROM THE FOOD CHAIN**
GAS-LIFT-FAULBEHÄLTER ZUM ANAEROBEN ABBAU VON ABFALL AUS DER NAHRUNGSKETTE
DIGESTEUR À POUSSÉE DE GAZ POUR LA DIGESTION ANAÉROBIE DE DÉCHETS PROVENANT DE LA CHAÎNE ALIMENTAIRE

(30) Priority: 06.02.2015 IT MI20150162
(43) Date of publication of application: 10.08.2016
(73) Proprietor: Natta, Francesco, 27010 Giussago (PV) (IT)
(72) Inventor: NATTA, Francesco, 27010 GIUSSAGO (PV) (IT); DONATI, Gianni, 27010 GIUSSAGO (PV) (IT)
(74) Representative: Bianchetti Bracco Minoja S.r.l.

(56) References cited:
- EP-A1- 2 578 558
- US-A1- 2002 192 809
- DATABASE WPI Week 201370 Thomson Scientific, London, GB; AN 2013-R03008 XP002745828, -& CN 202 945 239 U (CHEN) 22 May 2013 (2013-05-22)
- DATABASE WPI Week 201435 Thomson Scientific, London, GB; AN 2014-J36559 XP002745829, -& CN 103 667 030 A (NINGBO YINZHOU QIJIU INFORMATION TECHNOL.) 26 March 2014 (2014-03-26)

## Description

The object of the current invention is a new digester operating in continuous mode for the anaerobic digestion of waste coming from the cycle of production and consumption of foods for the purpose of producing fertilisers and biogas.

More particularly the invention relates to a new digester which uses the gas-lift technology for moving the mass during digestion and a series of conduits and internal compartmentalisations apt to guarantee the transfer of the mass from the centre to the periphery and/or vice versa and from the top downwards and vice versa and therefore ensure an efficient mixing during anaerobic digestion.

In this way the formation of dead zones, especially sediments and crusts on the surface of the digester is also avoided.

The use of the gas-lift technology allows for the efficient pumping of viscous fluids with high concentration of solids, and has a surprising notable effect of reducing of the viscosity of the process fluid during flow (thixotropy) and is also suitable for stripping ammonia from the fluid in gaseous phase.

In order to achieve these objectives the floor of the digester has been equipped with a series of cross-configuration horizontal conduits for conveying from the periphery to the centre; connected to a vertical conduit at the centre with a series of annular compartmentalisations which control the movement of the fluid from the centre to the periphery and vice versa.

Pumping by means of lifting gas can be performed with internal or external lift pipes or with a hybrid internal-external system according to the three but not limiting example applications.

### State of Innovation

Only recently has the use on an industrial scale of the waste coming from the cycle of production and consumption of foodstuffs become possible thanks to the availability of waste in concentrated form biological treated sludge, the separation of food waste contained in urban waste and to the collection of slurry from livestock farms.

This has enabled it to be transported, after eventual treatment, on to arable lands, even if they are far from the land from where they were produced and has required the installation of large systems for its treatment and transformation into fertilisers to be recycled in agriculture.

These plants perform the stabilisation of waste by means of systems of anaerobic digestion, through which the decomposable organic compounds, the source of unpleasant odours, are transformed into fertilisers and biogas.

This new approach and the wide availability of waste, has required the building of large-scale systems, no longer operating at the single farm level but on entire farming area or even on a regional scale, with the implementation of processes and technologies with high output and energy efficiency.

The most critical issue relate to the pumpability of the sludge which constitutes this said waste is the stirring and mixing in large digesters, often with volumes higher than 5000 m³. Moreover the presence of ammonia in the digester inhibits the completion of digestion, slowing down the process of anaerobic digestion and reducing the yields and therefore must be extracted from the viscous fluid during digestion.

Furthermore some waste, such as that deriving from the organic fraction of solid urban waste, has a content of fibres which, in the absence of good surface stirring, stratify and solidify on the waterline of the digesters, forming a solid "crust or cap", and are difficult to eliminate in large digestion tanks.

There are numerous patents related to digesters for the anaerobic digestion of similar substrates, but they are often aimed only at production of biogas, and not at the recovery of the nutrients for the means of fertiliser production.

The methods and the technologies of the digesters described to date, concentrate on the systems of stirring, while the stripping of the ammonia is often assigned to external units from the digester on a separate and fluid portion of the digestate.

Moreover there are no patents for the treatment of the fibrous crusts on the waterline of the digesters.

CA 1102019 discloses a combined heating and mixing system which uses a vertical conduit with sleeve placed at the centre of the digester equipped with the means for the pumping of the suspension from bottom upwards.

EP 0563434 proposes the mixing of the sludge in fermentation with a system of pipes placed on the base of the digester in the zones wherein the same sludge and the biogas are pumped in sequence.

GB 2457681 proposes as system of mixing a moveable arm rotating on the base of the digester actuated by a flow of gas.

US 4824571 divides the base of the digester into zones provided with systems of distribution of the gas to be activated in sequence.

US 6299774 operates essentially in batches with equipment of small dimensions stirred mechanically.

The previous patent MI2014A001362 of the Applicant, is aimed at the production of a digestate fertiliser and of an ammoniacal fertiliser using digesters in sequence operating in continuous mode. The transfer between the digesters is performed with gas lift pipes actuated with biogas and steam for the heating of the digesters and the stripping of the ammonia.

US 2002/192809 describes an anaerobic digestion apparatus with a vertical conduit in the center of the vessel and reintroduction of the produced biogas.

CN 202945239 discloses an anaerobic digester with a central draft tube and cross pipes on the base of the digester connected to the central conduit. The digesters are also equipped with auxiliary gas lift pipes for the mixing and the stripping of the ammonia yet nothing is said about the internal structure of the digester.

### Description of the invention

A new digester now disclosed, overcomes the limitations and disadvantages of the known aerobic digestion systems and completes the process described in the Applicant's previous patent MI2014A001362.

The new digester, the subject of this invention, allows the combination of the gas lift technology with a specific interior structure of the digester itself, using a synergic way in order to solve the problems highlighted above concerning the stirring and mixing.

The definition of this innovative digester with respect to the previous ones is due to various factors originating in the pilot testing, in the performing of technological tests and in the design phase of the industrial plant.

Firstly, given the great mass of waste to be treated in an industrial plant, at the design stage it is equipped with digesters having a volume of 5000 m³ with three digesters in series as in the patent MI2014A001362 of the Applicant.

Nevertheless it is unthinkable to consider dimensions of around 15000 m³ and over for each single digester with considerable problems of pumping and of mixing of the solids in the digester due to the high concentrations of total solids that are higher than 15%.

The technical tests by means of a pumping system with a gas-lift on the sludge produced by digestion had at the time (MI2014A001362) highlighted how the energy consumption of the gas lift was not higher than that of a pump for viscous fluids and how the working pressures of the gas were significantly lower, with the order of magnitude of the height and density of the aerated column.

A surprising reduction of 30% in the viscosity of the sludge treated with gas lift had also been noted.

This point has been analysed further in the laboratory by means of the rheological characterisation of various samples of sludge using apparatus for rotational rheometry (TA Instruments) at 25°C. It was seen that the fluid has high thixotropic characteristics with the dynamic viscosity which on the bilogarithmic scale drops linearly from 630 Pa .s to 1.1 Pa .s with a shear rate from 0.1 s-1 to 100 s-1.

At 55°C, the temperature at which anaerobic digestion is performed, these values drop further in an exponential manner.

The tests were therefore repeated on air lift in the process conditions, confirming the viscosity of the fluid of the same order of magnitude of those which occurred in the laboratory at 25°C with high shear rates.

This makes the gas lift system ideal for this type of fluid because it is able to offer efficient pumping even when it is in the form of sludge and contrarily is able to fluidify it at lower values than that of a corn syrup.

Thus a design of the industrial digester is required as it reduces the dead zones and maintains the fluid in movement with the properties generated in the gas-lift treatment.

A series of layouts were therefore designed for the interiors of the reactor so as to exploit these characteristics of the fluid all combined with the gas lift system.

Further features of the invention will be made clearer by the following detailed description, referred to its purely exemplary embodiments and therefore non-limiting, illustrated in the accompanying drawings, in which:

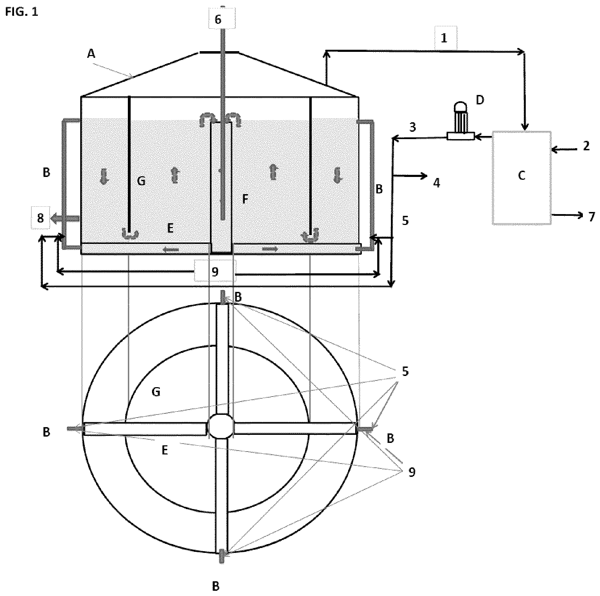

Figs. 1, 2 and 3 show three possible layouts of a digester according to the invention, in schematic vertical section and plan view respectively.

In all the layouts the gas lifts B are actuated using the biogas extracted from the digester A with the flow 1, after absorption of the ammonia with acid 2 in a conventional acid scrubber C and its compression in the compressor D.

The ammonium salt produced is sent with the flow 7 to storage.

The compressed biogas 5 with the flow 3 is in part sent to a gasometer with the flow 4 and in part sent to the gas lifts B.

In the gas lifts B steam 9 is also injected, generally available on the system, with the aim of supplying heat to the digester A and bringing it to the optimal temperature of 55°C to facilitate with the temperature and the induced vibrations the fluidification of the mass.

The feeding of the sludge to the digester A can be performed as indicated with the flow 6 or in different points of the digester, not shown in the drawings, so as to facilitate the gradual filling of the same during start-up.

The digestate produced with the flow 8 is extracted from the digester and sent to storage.

All the diagrams proposed with Figs. 1, 2 and 3 have in common:
- a vertical conduit F placed preferably in the centre of the digester;
- a cross of pipes E placed on the base of the digester and connected with the central conduit F in which the fluid is conveyed from the periphery to the centre or vice versa. These cross pipes can be four or more.
- Cylindrical compartments G divide the radial direction of the digester, some of which open upwards with an overflow onto the waterline and others downwards onto the base, in order to control the movement of the fluid from the top downwards and vice versa.

The number of these compartments can also vary in relation to the layout and to the dimensions of the digester as seen here below.

In particular Fig. 1 shows a layout which we will refer to as with external gas lifts because the movement of the fluid is performed with pipes B external to the digester and connected directly to the wall with base cross E and therefore with the vertical pipe F at the centre of the digester.

A single compartmentalisation of the digester G is provided, opening towards the base of the digester and the compartmentalisation is preferably done so as to divide the digester with equal areas of passage.

By sending the biogas 5 and the steam 9 just above the base of the four pipes B, aspiration of fluid is performed from the arms of the that cross E and the central pipe F and send it to the outer surface of the compartment from which it begins its descent to rise again in the internal compartment and overflow into the central conduit F as indicated by the arrows.

In this way a circular flow is created from the periphery to the centre and vice versa which involves the entire digester from the top downwards and vice versa with movement of the surface by the jet of the gas lifts and of the overflows.

As mentioned it is possible to place several arms on the cross E and several lifts B and it is also possible to increase the number of compartments. In this case it is preferable to have an odd number of compartmentalisation baffles (not including the central pipe).

By way of an example, in a digester having a total volume of 5000 m3 and a working volume of 4500 m3 fed with 4600 kg/h of sludge and with a flow of biogas of 3500 Nm3/h fed to the 4 external lift pipes, it is possible, with a power consumption of approximately 80 kW, to pump from the base to 11 m on the surface approximately 1700 m3/h of fluid and therefore with a characteristic time of mixing of around 3 h.

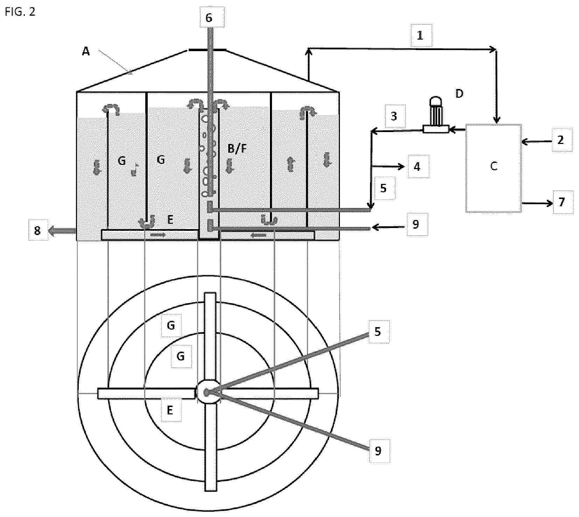

Fig. 2 shows a layout wherein the central pipe B/F is used as the only gas lift of the digester in which the compressed biogas 5 and the steam 9 (internal lift) are sent.

In this case the cross of pipes E, connected to the central pipe F, aspirates the fluid from the base of the peripheral layer and the digester is divided by an even number of compartmentalisation baffles G.

With the same dimensions of the digester, the previous examples' performances are similar but, due to the higher number of baffles, the speed of rise and descent in the open areas between the baffles are slightly higher: 16 m/h versus 11 m/h.

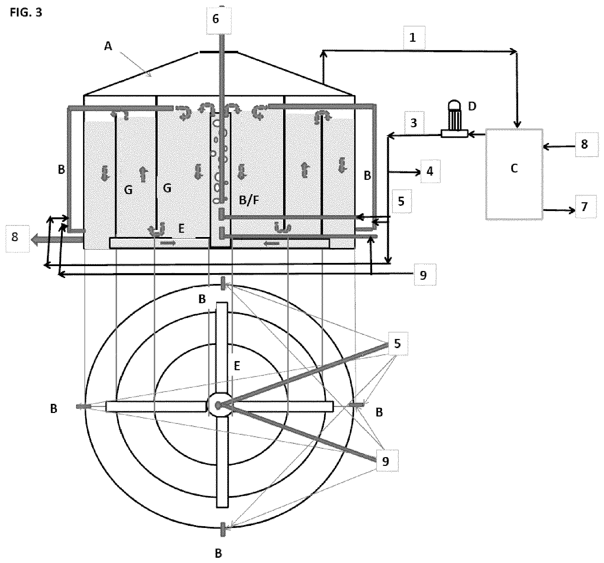

Fig. 3 shows a possible layout which we will call hybrid as provided with a single internal lift pipe B/F and external lift pipes B.

In this case the external lift pipes B aspirate directly from the base of the last compartment while the internal lift pipe B/F removes from the last compartment via the cross E. Both the internal and external lift pipes send the pumped fluid into the first compartment close to the pipe B/F.

It is possible in this case to operate independently the mode with, external lift pipe or only the internal lift pipe or with both lift pipes activated.

With the same flow rate of gas used, the performances are equivalent to those of the previous examples, without detriment to the fact that the increased number of compartments, increases the speeds of rise and descent of the fluids in the digester.

It was found that by increasing with the same system the flow of gas, the performances of the lift pipes increase and therefore the mixing in the reactors but also the energy consumption rates increase.

It is however, advisable to operate in the lift pipes with the speed of the gas with pipe empty comprised between 0.5 and 5 m/s and preferably between 1 and 3 m/s, depending on the need for stirring and above all for stripping of the ammonia.

It is however pointed out, that the distribution of the retention time in the digesters, such as those shown, is such as to limit the output of fresh material from the reactor.

To overcome this problem, the first industrial plant at the design stage has been built with three 5000 m³ reactors in series.

During operation it will be possible to evaluate the efficiency of the anaerobic digestion in the single reactors for future developments.

The digesters that are the object of the present invention, can operate, as mentioned, also with wastes containing in part fibrous fractions thanks to the intense surface wetting of the lift pipes and to the overflow which return the fibres towards the base, avoiding surface floating.

## Claims

1. Gas-lift digester (A) operating in continuous mode for the anaerobic digestion of waste coming from the cycle of production and consumption of foods for the purpose of producing fertilisers and biogas, where part of the biogas produced (5) is sent to at least one gas-lift pipe (B) of the digester, **characterised in that** it likewise comprises of:
- a vertical conduit (F) placed preferably in the centre of the digester (A);
- a cross of pipes (E) placed on the base of the digester and connected with the central conduit (F) for the conveying of the fluid from the periphery to the centre or vice versa;
- at least one cylindrical compartmentalisation baffle (G) of division in radial direction of the digester, open upwards with overflow onto the waterline or downwards onto the base, to control the orderly flow of fluid from the top downwards and vice versa and the movement on the surface and on the base of the digester.

2. Gas-lift digester according to claim 1, **characterised in that**, it provides a number of gas-lift pipes (B) external to the digester (A), equal in number to the number of pipes of the cross (E), where they are connected; and an odd number of compartmentalisation baffles (G), that alternatively open upwards with overflow on the waterline and downwards onto the base of the digester, the biogas (5) is sent to just above the base of the gas-lift pipes (B) creating an aspiration of the fluid by the arms of the cross (E) and by the central conduit (F) sending it to the surface of the external compartment wherefrom it starts its descent to rise again in the successive compartment and eventually overflow into the central conduit (F).

3. Gas-lift digester according to claim 1, **characterised in that**, it provides a single internal gas-lift pipe (B), coinciding with said central conduit (F), wherein the compressed biogas (5) is sent, and an even number of compartmentalisation baffles (G) alternatively open upwards with overflow on the waterline and downwards on the base of the digester, said cross of pipes (E), connected with the central pipe (F), sucking the fluid from the base of the peripheral compartment.

4. Gas-lift digester according to claim 3, **characterised in that**, it likewise provides a number of external gas-lift pipes (B) which aspirate directly from the base of the peripheral compartment, wherefrom the internal lift pipe (B/F) also aspirates via said cross (E).

5. Gas-lift digester according to claim 4, wherein both the internal lift pipe (B/F) and the external lift pipes (B) send the pumped fluid into the innermost compartment, close to the lift pipe (B/F).

6. Gas-lift digester according to claim 4 or 5, can operate only with external lifts (B) activated, or with only the internal lift (B/F) activated or with both external lifts and internal lift activated.

7. Gas-lift digester according to any one of the preceding claims, **characterised in that**, the said gas-lift pipes (B, B/N) steam (9) is also injected, with the aim of supplying heat to the digester (A) and bringing it to the optimal temperature of 55°C and of facilitating with the temperature and the vibrations caused the fluidification of the mass.

8. Gas-lift digester according to any one of the preceding claims, **characterised in that**, the supply of sludge to the digester is performed with a flow (6) through said central conduit (F).

9. Gas-lift digester according to any one of the preceding claims, wherein the said biogas (5) is extracted from the digester (A) with a flow (1) and sent to the gas-lift pipes (B, B/N), after absorption of the ammonia with acid (2) in an acid scrubber (C) and its compression in compressor (D).

10. Method for the anaerobic digestion of waste coming from the cycle of production and consumption of foods for the purpose of producing fertilisers and biogas by means of the gas-lift digester according to any one of the preceding claims.

## Patentansprüche

1. Gas-Lift-Faulbehälter (A), der im kontinuierlichen Modus zum anaeroben Abbau von Abfall, der aus dem Kreislauf von Nahrungsmittelherstellung und -verbrauch stammt, zum Zweck der Herstellung von Dünger und Biogas im Betrieb ist, wobei ein Teil des hergestellten Biogases (5) zu mindestens einem Gas-Lift-Rohr (B) des Faulbehälters geleitet wird, der **dadurch gekennzeichnet ist, dass** er ebenfalls umfasst:
- eine vertikale Leitung (F), die vorzugsweise in der Mitte des Faulbehälters (A) angeordnet ist;
- ein Rohrkreuz (E), das am Boden des Faulbehälters angeordnet ist und mit der Zentralleitung (F) verbunden ist, zum Befördern von Flüssigkeit aus dem Randbereich zur Mitte und umgekehrt;
- mindestens eine zylindrische Kompartimentierungstrennwand (G) zur Aufteilung des Faulbehälters in radialer Richtung, die nach oben mit Überfluss auf die Wasserlinie oder nach unten zum Boden geöffnet ist, um den ordnungsgerechten Fluss der Flüssigkeit von oben nach unten und umgekehrt und die Bewegung auf der Oberfläche und auf dem Boden des Faulbehälters zu kontrollieren.

2. Gas-Lift-Faulbehälter nach Anspruch 1, **dadurch gekennzeichnet, dass** er die gleiche Anzahl an Gas-Lift-Rohren (B), die extern vom Faulbehälter (A) sind, bereitstellt wie die Anzahl an Rohren des Kreuzes (E), wo sie miteinander verbunden sind; und eine ungerade Anzahl an Kompartimentierungstrennwänden (G), die sich abwechselnd nach oben mit Überfluss auf die Wasserlinie und nach unten zum Boden des Faulbehälters öffnen, das Biogas (5) bis gerade über den Boden der Gas-Lift-Rohre (B) geleitet wird, wodurch ein Ansaugen der Flüssigkeit durch die Arme des Kreuzes (E) und durch die Zentralleitung (F) erzeugt wird, wodurch die Flüssigkeit zur Oberfläche des externen Kompartiments geleitet wird, von wo sie ihren Abfluss bis zum Wiederaufstieg im folgenden Kompartiment beginnt und schließlich in die Zentralleitung (F) überfließt.

3. Gas-Lift-Faulbehälter nach Anspruch 1, **dadurch gekennzeichnet, dass** er ein einzelnes internes Gas-Lift-Rohr (B), das mit der Zentralleitung (F) zusammenfällt, wobei das komprimierte Biogas (5) geleitet wird, und eine gerade Anzahl an Kompartimentierungstrennwänden (G) bereitstellt, die sich abwechselnd nach oben mit Überfluss auf die Wasserlinie und nach unten zum Boden des Faulbehälters öffnen, wobei das Rohrkreuz (E), das mit der Zentralleitung (F) verbunden ist, die Flüssigkeit vom Boden des peripheren Kompartiments aufsaugt.

4. Gas-Lift-Faulbehälter nach Anspruch 3, **dadurch gekennzeichnet, dass** er ebenfalls eine Anzahl an externen Gas-Lift-Rohren-(B) bereitstellt, die direkt vom Boden des peripheren Kompartiments absaugen, von wo das interne Lift-Rohr (B/F) auch über das Kreuz (E) absaugt.

5. Gas-Lift-Faulbehälter nach Anspruch 4, wobei sowohl das interne Lift-Rohr (B/F) als auch die externen Lift-Rohre (B) die gepumpte Flüssigkeit in das innerste Kompartiment leiten, das sich nahe des Lift-Rohrs (B/F) befindet.

6. Gas-Lift-Faulbehälter nach Anspruch 4 oder 5, der nur mit aktivierten externen Lifts (B) oder nur mit dem aktivierten internen Lift (B/F) oder mit sowohl aktivierten externen Lifts und aktiviertem internen Lift betrieben werden kann.

7. Gas-Lift-Faulbehälter nach einem der vorangehenden Ansprüche, **dadurch gekennzeichnet, dass** in die Gas-Lift-Rohre (B, B/N) auch Dampf (9) injiziert wird mit dem Ziel, dass der Gas-Lift-Faulbehälter (A) mit Wärme versorgt wird und auf die optimale Temperatur von 55°C gebracht wird und dass mit der Temperatur und den verursachten Vibrationen die Verflüssigung der Masse ermöglicht wird.

8. Gas-Lift-Faulbehälter nach einem der vorangehenden Ansprüche, **dadurch gekennzeichnet, dass** die Zufuhr von Schlamm in den Faulbehälter mit einem Durchfluss (6) durch die Zentralleitung (F) bewerkstelligt wird.

9. Gas-Lift-Faulbehälter nach einem der vorangehenden Ansprüche, wobei das Biogas (5) aus dem Faulbehälter (A) mit einem Durchfluss (1) extrahiert wird und nach der Absorption von Ammoniak mit Säure (2) in einem Säurewäscher (C) und seiner Verdichtung im Kompressor (D) zu den Gas-Lift-Rohren (B, B/N) geleitet wird.

10. Verfahren zum anaeroben Abbau von Abfall, der aus dem Kreislauf von Nahrungsmittelherstellung und -verbrauch stammt, zum Zweck der Herstellung von Dünger und Biogas mittels des Gas-Lift-Faulbehälters nach einem der vorangehenden Ansprüche.

## Revendications

1. Digesteur à poussée de gaz (A) fonctionnant en mode continu destiné à la digestion anaérobie de déchets provenant du cycle de production et de consommation d'aliments aux fins de produire des engrais et du biogaz, où une partie du biogaz produit (5) est envoyée à au moins une canalisation à poussée de gaz (B) du digesteur, **caractérisé en ce que** de la même manière il est composé :
- d'un conduit vertical (F) placé préférablement dans le centre du digesteur (A) ;
- d'une croix de canalisation (E) placée sur la base du digesteur et connectée au conduit central (F) pour le transport du fluide depuis la périphérie vers le centre ou vice versa ;
- d'au moins une chicane de compartimentalisation cylindrique (G) de division dans le sens radial du digesteur, ouverte vers le haut avec un trop-plein sur la ligne d'eau ou vers le bas sur la base, pour réguler de manière ordonnée l'écoulement du fluide depuis le haut vers le bas et vice versa et le mouvement sur la surface et sur la base du digesteur.

2. Digesteur à poussée de gaz selon la revendication 1, **caractérisé en ce que**, est prévu un nombre de canalisations à poussée de gaz (B) externes au digesteur (A), égales en nombre au nombre de canalisations de la croix (E), où elles sont connectées ; et un nombre impair de chicanes de compartimentalisation (G), qui s'ouvrent alternativement vers le haut avec le trop-plein sur la ligne d'eau et vers le bas sur la base du digesteur, le biogaz (5) est envoyé juste au-dessus de la base des canalisations à poussée de gaz (B) créant une aspiration du fluide par les bras de la croix (E) et par le conduit central (F) l'envoyant à la surface du compartiment externe depuis lequel il commence sa descente pour s'élever à nouveau dans le compartiment successif et éventuellement se trouver en trop-plein dans le conduit central (F).

3. Digesteur à poussée de gaz selon la revendication 1, **caractérisé en ce que**, est prévue une canalisation unique interne à poussée de gaz (B), coïncidant avec ledit conduit central (F), le biogaz comprimé (5) étant envoyé, et un nombre pair de chicanes de compartimentalisation (G) s'ouvrant alternativement vers le haut avec le trop-plein sur la ligne d'eau et vers le bas sur la base du digesteur, ladite croix de canalisation (E), connectée à la canalisation centrale (F), aspirant le fluide depuis la base du compartiment périphérique.

4. Digesteur à poussée de gaz selon la revendication 3, **caractérisé en ce que**, est prévu de la même manière un nombre de canalisations externes à poussée de gaz (B) qui aspirent directement depuis la base du compartiment périphérique, depuis lequel la canalisation interne d'élévation (B/F) aspire également par l'intermédiaire de ladite croix (E).

5. Digesteur à poussée de gaz selon la revendication 4, dans lequel à la fois la canalisation interne d'élévation (B/F) et les canalisations externes d'élévation (B) envoient le fluide pompé dans le compartiment le plus interne, proche de la canalisation d'élévation (B/F).

6. Digesteur à poussée de gaz selon la revendication 4 ou 5, pouvant fonctionner uniquement avec les élévations externes (B) activées, ou avec uniquement l'élévation interne (B/F) activée ou avec à la fois les élévations externes et l'élévation interne activées.

7. Digesteur à poussée de gaz selon l'une quelconque des revendications précédentes, **caractérisé en ce que**, ladite vapeur (9) de canalisations à poussée de gaz (B, B/N) est également injectée, dans le but d'alimenter de la chaleur au digesteur (A) et le portant à la température optimale de 55°C et facilitant la température et les vibrations provoquées par la fluidification de la masse.

8. Digesteur à poussée de gaz selon l'une quelconque des revendications précédentes, **caractérisé en ce que**, l'alimentation de boues au digesteur est exécutée avec un écoulement (6) à travers ledit conduit central (F).

9. Digesteur à poussée de gaz selon l'une quelconque des revendications précédentes, dans lequel ledit biogaz (5) est extrait du digesteur (A) avec un écoulement (1) et envoyé vers les canalisations à poussée de gaz (B, B/N), après l'absorption de l'ammoniac avec de l'acide (2) dans un épurateur d'acide (C) et sa compression dans le compresseur (D).

10. Procédé de digestion anaérobie de déchets provenant du cycle de production et de consommation d'aliments aux fins de produire des engrais et du biogaz par l'intermédiaire du digesteur à poussée de gaz selon l'une quelconque des revendications précédentes.
